(19) Europäisches Patentamt
European Patent Office
Office européen des brevets

(11) Veröffentlichungsnummer: **0 017 116**
**B1**

(12) **EUROPÄISCHE PATENTSCHRIFT**

(45) Veröffentlichungstag der Patentschrift: **17.02.82**

(51) Int. Cl.³: **C 07 C 7/08**, C 07 C 11/12

(21) Anmeldenummer: **80101527.2**

(22) Anmeldetag: **22.03.80**

(54) **Verfahren zur Gewinnung eines konjugierten Diolefins aus einem C 4- oder C 5-Kohlenwasserstoffgemisch.**

(30) Priorität: **23.03.79 DE 2911393**

(43) Veröffentlichungstag der Anmeldung: **15.10.80 Patentblatt 80/21**

(45) Bekanntmachung des Hinweises auf die Patenterteilung: **17.02.82 Patentblatt 82/7**

(84) Benannte Vertragsstaaten: **AT BE CH DE FR GB IT NL SE**

(56) Entgegenhaltungen:
**DE-B2-2 521 965**
**US-A-3 317 627**
**US-A-3 769 217**

(73) Patentinhaber: **BASF Aktiengesellschaft, Carl-Bosch-Strasse 38, D-6700 Ludwigshafen (DE)**

(72) Erfinder: **Volkamer, Klaus, Dr., Heidelberger Ring 21, D-6710 Frankenthal (DE)**
Erfinder: **Broellos, Klaus, Dr., Floriansring 7, D-6701 Seeheim (DE)**
Erfinder: **Lindner, Alfred, Dr., Ringstrasse 22, D-6712 Bobenheim-Roxheim 1 (DE)**
Erfinder: **Wagner, Ulrich, Dr., Knospstrasse 7, D-6703 Limburgerhof (DE)**
Erfinder: **Weitz, Hans-Martin, Dr., Auf dem Koeppel 40, D-6702 Bad Duerkheim 1 (DE)**
Erfinder: **Schneider, Klaus-Juergen, Dr., Triftbrunnenweg 16, D-6730 Neustadt 19 (DE)**

EP 0 017 116 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Gewinnung eines konjugierten Diolefins aus einem $C_4$- oder $C_5$-Kohlenwasserstoffgemisch

Die vorliegende Erfindung betrifft ein Verfahren zur Gewinnung eines konjugierten Diolefins aus einem $C_4$- oder $C_5$-Kohlenwasserstoffgemisch durch extraktive Destillation mit Hilfe eines selektiven Lösungsmittels.

Die extraktive Destillation ist ein bekanntes Verfahren zur Trennung von Gemischen, die durch übliche fraktionierte Destillation nicht leicht trennbar sind, z. B. wenn die zu trennenden Komponenten ein Azeotrop bilden oder geringe Unterschiede in den relativen Flüchtigkeiten besitzen. Bei der extraktiven Destillation wird in die Destillationskolonne eine solche Menge eines relativ schwer flüchtigen Lösungsmittels eingeführt, daß die Unterschiede in den relativen Flüchtigkeiten der zu trennenden Komponenten erhöht werden und damit eine destillative Trennung ermöglicht wird. Typische Anwendungsbeispiele für die extraktive Destillation finden sich beispielsweise in C. S. Robinson et al »Elements of Fractional Distallation« 4. Auflage McGraw-Hill Book Company, Inc., New York, (1959), Seite 291.

Es ist bekannt, z. B. aus der DE-AS 1 568 902, DE-PS 1 163 795 oder aus The Societ Chemical Industry, Nr. 11, November 1971, Seiten 719 bis 723, konjugierte Diolefine aus einem $C_4$- oder $C_5$-Kohlenwasserstoffgemisch durch extraktive Destillation unter Verwendung eines selektiven Lösungsmittels zu gewinnen. Die selektiven Lösungsmittel können weitgehend wasserfrei verwendet werden. Bei dieser Arbeitsweise, die besonders bei hydrolyseempfindlichen Lösungsmitteln angewendet wird, ist jedoch im allgemeinen die $C_4$- oder $C_5$-Kohlenwasserstoff-Selektivität nicht zufriedenstellend. Den selektiven Lösungsmitteln ist daher zur Erhöhung der Selektivität und zur Erniedrigung des Siedepunktes Wasser zugemischt worden. Die Wasserzugabe zum selektiven Lösungsmittel hat jedoch den Nachteil, daß die Löslichkeit der $C_4$- oder $C_5$-Kohlenwasserstoffe im selektiven Lösungsmittel erniedrigt wird, so daß die Menge an umlaufenden selektivem Lösungsmittel in der Extraktionsanlage entsprechend erhöht ist.

Aus der DE-AS 2 521 965 ist weiter ein Verfahren zur Gewinnung von Butadien aus einem dieses enthaltenden $C_4$-Kohlenwasserstoffgemisch bekannt, bei dem zunächst Isobutylen in Anwesenheit eines sauren Ionenaustauscherharzes mit Methanol veräthert und der gebildete Methyl-tert.-butyläther durch Destillation vom $C_4$-Kohlenwasserstoffgemisch abgetrennt wird. Das nach der Destillation erhaltene $C_4$-Kohlenwasserstoffgemisch wird anschließend zur Verätherung der Acetylenverbindungen wie Propyn, Vinylacetylen und 1-Butyn, die in sehr geringen Mengen im $C_4$-Kohlenwasserstoffgemisch enthalten sind, in einer zweiten Verätherungsstufe in Gegenwart eines sauren Ionenaustauscherharzes, das Quecksilberionen enthält, mit Methanol unter Bildung der Vinyläther umgesetzt. Das aus der zweiten Verätherungsstufe erhaltene $C_4$-Kohlenwasserstoffgemisch, welches nur sehr geringe Mengen Äther enthält, wird anschließend zur Abtrennung des Butadiens einer extraktiven Destillation unterworfen. Dieses Verfahren ist durch die Vorschaltung von zwei Verätherungsstufen sehr aufwendig. Außerdem ist in der zweiten Verätherungsstufe die Verwendung von Quecksilbersalzen erforderlich, die erhebliche Umweltschutzprobleme hervorrufen können.

Die vorliegende Erfindung soll nun eine Verbesserung der Arbeitsweise und Wirtschaftlichkeit der bekannten Verfahren bewirken.

Es wurde nun ein vorteilhaftes Verfahren gefunden zur Gewinnung eines konjugierten Diolefins aus einem dieses enthaltenden $C_4$- oder $C_5$-Kohlenwasserstoffgemisch durch ein- oder mehrstufige extraktive Destillation mit einem selektiven Lösungsmittel, welches dadurch gekennzeichnet ist, daß man als selektives Lösungsmittel eine Lösungsmittelmischung verwendet, die

a) 50 bis 98 Gewichtsprozent eines N-alkylsubstituierten niederen aliphatischen Säureamids oder eines N-alkylsubstituierten alicyclischen Säureamids mit 5 Ringgliedern und

b) 2 bis 50 Gewichtsprozent eines
   - aliphatischen Äthers mit einem Siedepunkt im Bereich von 30 bis 200°C der allgemeinen Formel $R-O-R'$, worin R ein Kohlenwasserstoffrest mit 1 bis 6 Kohlenstoffatomen und R' ein Kohlenwasserstoffrest mit 2 bis 6 Kohlenstoffatomen ist, oder der allgemeinen Formel

     $$R^1-[OCH_2-CHR-]_nO-R^2,$$

     in der R ein Wasserstoffatom oder einen $CH_3$-Rest, $R^1$ und $R^2$ jeweils einen Kohlenwasserstoffrest mit 1 bis 5 Kohlenstoffatomen und n eine ganze Zahl von 1 bis 3 bedeuten, und $R^1$ darüber hinaus für ein Wasserstoffatom stehen kann, bzw.
   - alicyclischen Äthers mit einem Siedepunkt im Bereich von 50°C bis 125°C

enthält.

Die Löslichkeit der $C_4$- oder $C_5$-Kohlenwasserstoffe in der erfindungsgemäß als selektives Lösungsmittel zu verwendenden Lösungsmittelmischung ist gegenüber den Lösungsmitteln der bekannten Verfahren bei vergleichbar guter $C_4$- oder $C_5$-Kohlenwasserstoff-Selektivität wesentlich erhöht, so daß die Menge an umlaufendem selektivem Lösungsmittel in der Extraktionsanlage zur Gewinnung des konjugierten Diolefins stark erniedrigt werden kann. Dies hat insbesondere eine starke Senkung der Investitionskosten

für die Extraktionsanlage und der Verbräuche an Dampf und elektrischer Energie zur Folge. Außerdem weist die erfindungsgemäß zu verwendende Lösungsmittelmischung eine geringere Viskosität, eine geringere Verdampfungswärme und eine geringere spezifische Wärme als die bekannten selektiven Lösungsmittel mit vergleichbar guter $C_4$- oder $C_5$-Kohlenwasserstoff-Selektivität auf. Die geringere Viskosität der erfindungsgemäß zu verwendenden Lösungsmittelmischung bewirkt einen besseren Bodenwirkungsgrad in den extraktiven Destillationskolonnen, während durch die geringere Verdampfungswärme und geringere spezifische Wärme zusätzlich Energie eingespart werden kann.

Bei Verwendung einer Mischung eines der relativ hoch siedenden Lösungsmittel gemäß der vorstehenden Ziffer a) mit einem niedriger als dieses siedenden Lösungsmittel gemäß vorstehender Ziffer b), z. B. einem niedriger als 150°C, vorzugsweise niedriger als 125°C, siedenden Lösungsmittel gemäß vorstehender Ziffer b) wird zusätzlich der Vorteil erhalten, daß die beispielsweise als Ausgaser oder als Lösungsmittelabstreifer betriebene Lösungsmittelwiedergewinnungszone der Extraktionsanlage zur Gewinnung des konjugierten Diolefins bei jeweils gleicher Sumpftemperatur bei höherem Druck als mit den reinen unter Ziffer a) genannten Lösungsmitteln nach den bekannten Verfahren betrieben werden kann. Dies hat beispielsweise den Vorteil, daß die in der Lösungsmittelgewinnungszone erhaltenen ausgegasten Kohlenwasserstoffe auf Grund des höheren Drucks ohne Zwischenschaltung eines Kompressors in nachgeschaltete, unter erhöhtem Druck betriebene Zonen gefördert werden können. Ein anderer Vorteil der Verwendung einer Mischung eines der relativ hoch siedenden Lösungsmittel gemäß der vorstehenden Ziffer a) mit einem niedriger als dieses siedenden Lösungsmittel gemäß vorstehender Ziffer b) besteht darin, daß die z. B. als Ausgaser oder als Lösungsmittelabstreifer betriebene Lösungsmittelwiedergewinnungszone der Extraktionsanlage bei Anwendung des gleichen Drucks wie nach den bekannten Verfahren bei einer niedrigeren Sumpftemperatur betrieben werden kann, so daß eine Verschmutzung der Extraktionsanlage durch Polymerbildung leichter vermieden werden kann.

Für das Verfahren nach der Erfindung wird eine Lösungsmittelmischung verwendet, die

a) 50 bis 98 Gewichtsprozent eines N-alkylsubstituierten niederen aliphatischen Säureamids oder eines N-alkylsubstituierten alicyclischen Säureamids mit 5 Ringgliedern und
b) 2 bis 50 Gewichtsprozent eines
   - aliphatischen Äthers mit einem Siedepunkt im Bereich von 30 bis 200°C der allgemeinen Formel $R-O-R'$, worin R ein Kohlenwasserstoffrest mit 1 bis 6 Kohlenstoffatomen und R′ ein Kohlenwasserstoffrest mit 2 bis 6 Kohlenstoffatomen ist, oder der allgemeinen Formel

     $$R^1-[OCH_2-CHR-]_nO-R^2,$$

     in der R ein Wasserstoffatom oder einen $CH_3$-Rest, $R^1$ und $R^2$ jeweils einen Kohlenwasserstoffrest mit 1 bis 5 Kohlenstoffatomen und n eine ganze Zahl von 1 bis 3 bedeuten, und $R^1$ darüber hinaus für ein Wasserstoffatom stehen kann, bzw.
   - alicyclischen Äthers mit einem Siedepunkt im Bereich von 50°C bis 125°C enthält.

Geeignete N-alkylsubstituierte niedere aliphatische Säureamide sind z. B. Dimethylformamid, Diäthylformamid, Dimethylacetamid, Diäthylacetamid, Formylmorpholin, Als N-alkylsubstituierte alicyclische Säureamide (Lactame) kommen z. B. N-Alkylpyrrolidone, insbesondere N-Methylpyrrolidon, in Betracht. Im allgemeinen werden N-alkylsubstituierte niedere aliphatische Säureamide oder N-alkylsubstituierte alicyclische Säureamide mit Siedepunkten im Bereich von 150°C bis 260°C, vorzugsweise im Bereich von 150°C bis 210°C, verwendet. Mit besonderem Vorteil werden von den Lösungsmitteln gemäß vorstehender Ziffer a) Dimethylformamid und insbesondere N-Methylpyrrolidon verwendet.

Die erfindungsgemäß zu verwendende Lösungsmittelmischung enthält 2 bis 50 Gewichtsprozent, vorzugsweise 3 bis 20 Gewichtsprozent, an einem der Lösungsmittel gemäß vorstehender Ziffer b). Entsprechend weist die Lösungsmittelmischung einen Gehalt von 50 bis 98 Gewichtsprozent, vorzugsweise 80 bis 97 Gewichtsprozent, an einem der Lösungsmittel gemäß vorstehender Ziffer a) auf. Bei der Verwendung einer N-Methylpyrrolidon enthaltenden Lösungsmittelmischung weist diese Mischung mit besonderem Vorteil einen Gehalt an einem Lösungsmittel gemäß vorstehender Ziffer b) von 7 bis 17 Gewichtsprozent auf.

Als aliphatische Äther werden symmetrische oder unsymmetrische Äther der allgemeinen Formel $R-O-R'$ verwendet, in denen der aliphatische Rest R ein Kohlenwasserstoffrest mit 1 bis 6, vorzugsweise 1 bis 5, insbesondere 1 bis 4 Kohlenstoffatomen und der aliphatische Rest R′ ein Kohlenwasserstoffrest mit 2 bis 6, vorzugsweise 2 bis 5 Kohlenstoffatomen ist. Die Gesamtzahl der Kohlenstoffatome beider Kohlenwasserstoffreste beträgt zweckmäßig 4 bis 12, vorzugsweise 5 bis 10.

Geeignete Reste R und R′ sind z. B. der Äthyl-, n-Propyl-, Isopropyl-, n-Butyl-, Isobutyl-, tert.-Butyl-Rest, die Amyl-Reste, Hexyl-Reste, wobei R zusätzlich einen Methyl-Rest bedeuten kann. Geeignete Äther sind beispielsweise symmetrische Äther, wie Diäthyläther, Di-n-propyläther, Diisopropyläther, Di-n-butyl-äther, Diisobutyläther, Di-n-amyläther, Diisoamyläther sowie vorzugsweise unsymmetrische Äther, wie Methyl-n-butyläther, Methyl-tert.-butyläther, Äthyl-tert.-butyläther, n-Propyl-tert.-butyläther, Isopropyl-tert.-butyläther, Isobutyl-tert.-butyläther, n-Butyl-tert.-butyläther, Methyl-tert.-amyläther, Äthyl-tert.-amyläther, n-Propyl-tert.-amyläther, Isopropyl-tert.-amyläther, n-Butyl-tert.-amyläther, Isobutyl-tert.-amyläther, Methyl-isopropyläther, Äthyl-isopropyläther, n-Propyl-isopropyläther, n-Butyl-isopropyläther, Isobutyl-isopropyläther, Äthyl-n-butyläther. Besonders vorteilhaft werden von den unsymmetrischen Äthern solche verwendet, in denen ein Rest eine tert.-Amyl-, bzw. Isopropyl- oder insbesondere eine tert.-Butyl-Gruppe ist.

Weiter werden als aliphatische Äther die Monoalkyläther und vorzugsweise Dialkyläther der allgemeinen Formel

$$R^1-[OCH_2-CHR]_nO-R^2$$

verwendet, in der R ein Wasserstoffatom oder einen $CH_3$-Rest, $R^1$ und $R^2$ jeweils einen Kohlenwasserstoffrest mit 1 bis 5, vorzugsweise 1 bis 4 Kohlenstoffatomen, und n eine ganze Zahl von 1 bis 3, vorzugsweise 1 bis 2 bedeuten und $R^1$ darüber hinaus für ein Wasserstoffatom stehen kann. Beispiele für Dialkyläther von zweiwertigen Alkoholen sind Äthylenglykol-dimethyläther, -diäthyläther, -di-n-propyläther, -diisopropyläther, -diisobutyläther, -methylisopropyläther, -methyl-tert.-butyläther, Diäthylenglykoldimethyläther, -methylisopropyläther, 1,2-Propylenglykol-dimethyläther, 1,4-Butandiol-dimethyläther. Geeignete Monoalkyläther von zweiwertigen Alkoholen sind z. B. Äthylenglykol-monomethyläther, -monoäthyläther, -mono-n-propyläther, -monoisopropyläther, -mono-n-butyläther, -monoisobutyläther, Diäthylglykolmonomethyläther, -monoäthyläther.

Als alicyclische Äther, d. h. cyclischer Äther, die außer Sauerstoffatomen keine weiteren Heteroatome enthalten, kommen beispielsweise 2-Methyltetrahydrofuran, 3-Methyltetrahydrofuran, Tetrahydropyran, 1,4-Dioxan oder vorzugsweise Tetrahydrofuran in Betracht.

Die erfindungsgemäß zu verwendende Lösungsmittelmischung kann einen geringen Wassergehalt, z. B. bis zu 10 Gewichtsprozent, aufweisen. Zweckmäßig wird jedoch der Wassergehalt auf höchstens 5 Gewichtsprozent, vorzugsweise höchstens 3 Gewichtsprozent, bezogen auf die Lösungsmittelmischung begrenzt. Es kann jedoch auch vorteilhaft sein, im wesentlichen wasserfrei, d. h. mit einem Wassergehalt von höchstens 1 Gewichtsprozent, vorzugsweise höchstens 0,5 Gewichtsprozent, insbesondere höchstens 0,1 Gewichtsprozent, bezogen auf die Lösungsmittelmischung, zu arbeiten.

Die Gewinnung eines konjugierten Diolefins aus einem dieses enthaltenden $C_4$- oder $C_5$-Kohlenwasserstoffgemisch unter Verwendung der erfindungsgemäß als selektives Lösungsmittel einzusetzenden Lösungsmittelmischung erfolgt in an sich bekannter Weise (vgl. z. B. DE-PS 1 184 334, DE-AS 1 568 876, DE-AS 1 568 902) durch ein- oder mehrstufige, zweckmäßig ein- oder zweistufige extraktive Destillation. Die Gewinnung der konjugierten Diolefine, wie 1,3-Butadien, Isopren und 1,3-Pentadien aus der $C_4$- oder $C_5$-Kohlenwasserstoffmischung erfolgt beispielsweise in der Weise, daß das $C_4$- oder $C_5$-Kohlenwasserstoffgemisch, welches löslichere Kohlenwasserstoffe als das konjugierte Diolefin und weniger lösliche Kohlenwasserstoffe als das konjugierte Diolefin enthält, einer extraktiven Destillation mit dem erfindungsgemäß zu verwendenden Lösungsmittelgemisch unterworfen wird, wobei ein die weniger löslichen Kohlenwasserstoffe enthaltendes Destillat und ein das konjugierte Diolefin und die löslicheren Kohlenwasserstoffe, und das selektive Lösungsmittel enthaltender Extrakt abgetrennt werden. Aus dem Extrakt kann das konjugierte Diolefin als Rohprodukt isoliert werden, das für manche Verwendungszwecke eine ausreichende Reinheit aufweist, das jedoch auch noch weiteren Reinigungsoperationen, z. B. einer fraktionierten Destillation, unterworfen werden kann. Vorteilhaft wird das konjugierte Diolefin jedoch unter Anwendung von zwei hintereinandergeschalteten extraktiven Destillationsstufen unter Verwendung der erfindungsgemäß zu verwendenden Lösungsmittelmischung gewonnen.

Hierbei werden beispielsweise — wie bereits vorstehend beschrieben — in der ersten Stufe der extraktiven Destillation ein die weniger löslichen Kohlenwasserstoffe enthaltendes Destillat und ein das konjugierte Diolefin und die löslicheren Kohlenwasserstoffe und das selektive Lösungsmittel enthaltender Extrakt abgetrennt. Dieser Extrakt wird vom selektiven Lösungsmittel befreit, wobei ein Gemisch aus dem konjugierten Diolefin und den löslicheren Kohlenwasserstoffen erhalten wird. Dieses Gemisch wird einer zweiten extraktiven Destillation mit dem selektiven Lösungsmittel unterworfen, wobei das konjugierte Diolefin als Destillat und ein Extrakt erhalten werden, der die löslicheren Kohlenwasserstoffe und das selektive Lösungsmittel enthält. Der erhaltene Extrakt wird anschließend vom selektiven Lösungsmittel befreit, wobei ein die löslicheren Kohlenwasserstoffe enthaltender Kohlenwasserstoffstrom erhalten wird.

Als konjugierte Diolefine enthaltendes Kohlenwasserstoffgemisch, das als Ausgangsgemisch für das Verfahren der vorliegenden Erfindung geeignet ist, kann eine $C_4$- oder $C_5$-Fraktion, die durch thermisches Kracken einer Petroleumfraktion (z. B. LPG, Naphtha usw.) erhalten wurde, eine durch Dehydrierung von n-Butan und/oder n-Buten erhaltene Butadien enthaltende Fraktion und eine durch Dehydrierung von Isopentan und/oder Isoamylen erhaltene Isopren enthaltende Fraktion verwendet werden. Im allgemeinen enthält das $C_4$-Kohlen-

wasserstoffgemisch 1,3-Butadien als konjugiertes Diolefin, Butane, n-Buten, Isobuten, Vinylacetylen, Äthylacetylen, 1,2-Butadien und gegebenenfalls geringe Mengen $C_3$- und/oder $C_5$-Kohlenwasserstoffe. Das $C_5$-Kohlenwasserstoffgemisch enthält in der Regel Isopren, trans- und cis-1,3-Pentadien und Cyclopentadien als konjugierte Diolefine sowie Pentane, n-Pentene, Isoamylen, Cyclopenten, höhere Acetylene.

Beispielsweise ergibt die extraktive Destillation einer $C_4$-Fraktion zunächst ein die Butane und Butene enthaltendes Destillat sowie einen 1,3-Butadien, Äthylacetylen, Vinylacetylen und 1,2-Butadien enthaltenden Extrakt, der, wenn er einer weiteren extraktiven Destillation unterworfen wird, als Destillat 1,3-Butadien liefert, während der Extrakt Äthylacetylen, Vinylacetylen und 1,2-Butadien enthält. Aus dem Äthylacetylen, Vinylacetylen und 1,2-Butadien enthaltenden Extrakt werden diese Kohlenwasserstoffe in einem Ausgaser abgetrennt und das entgaste Lösungsmittel in die extraktive Destillation zurückgeführt. Das als Destillat erhaltene 1,3-Butadien kann anschließend zur Abtrennung von gegebenenfalls noch vorhandenen sehr geringen Mengen von $C_3$- und/oder $C_5$-Kohlenwasserstoffen einer fraktionierten Destillation unterworfen werden.

Die nachstehenden Beispiele dienen der weiteren Erläuterung der Erfindung:

## Beispiel 1

Beispiel 1 veranschaulicht die einstufige extraktive Destillation eines $C_4$-Kohlenwasserstoffgemisches.

Einer bei 1 bar und 15°C betriebenen Füllkörperkolonne mit 25 mm Innendurchmesser und 2,50 m Höhe werden am Sumpf 0,858 kg/h $C_4$-Kohlenwasserstoffgemisch der folgenden Zusammensetzung zugeführt:

| Zusammensetzung | Gew.-% |
|---|---|
| i-Butan | 1,33 |
| n-Butan | 4,44 |
| Buten-1 | 11,65 |
| i-Buten | 28,21 |
| Buten-2-trans | 7,28 |
| Buten-2-cis | 4,45 |
| Butadien-1,3 | 41,98 |
| Butadien-1,2 | 0,31 |
| Äthylacetylen | 0,24 |
| Vinylacetylen | 0,11 |

Am Kopf der Kolonne werden 4,60 kg/h im Kreise geführtes selektives Lösungsmittel von 15°C aus 90 Gewichtsprozent N-Methylpyrrolidon und 10 Gewichtsprozent Methyl-tert.-butyläther zugegeben. 0,418 kg/h Raffinat mit einem Gehalt an Butadien-1,3 von 7,89 Gewichtsprozent und an Buten-2-cis (Schlüsselkomponente für die Trennung) von 4,78 Gewichtsprozent werden am Kopf der Kolonne gasförmig abgezogen. Butadien-1,2 und die $C_4$-Acetylene (Äthylacetylen, Vinylacetylen) sind im Rafinat nicht mehr nachweisbar. Am Sumpf der Kolonne wird ein die leichter löslichen Kohlenwasserstoffe enthaltender Extrakt abgezogen, der zur weiteren Trennung und Ausgasung dem Kopf einer nachgeschalteten Kolonne mit 10 Glockenböden zugeführt wird, deren Sumpftemperatur auf der Siedetemperatur von 130 bis 140°C gehalten wird. Vom Sumpf der Glockenbodenkolonne wird das weitgehend von den Kohlenwasserstoffen befreite selektive Lösungsmittel nach Abkühlung auf den Kopf der Füllkörperkolonne zurückgeführt. In der Mitte der Glockenbodenkolonne werden 0,440 kg/h Rohbutadien mit einem Butadien-1,3-Gehalt von 74,55 Gewichtsprozent und einem Buten-2-cis-Gehalt von 4,09 Gewichtsprozent abgezogen. Die am Kopf der Glockenbodenkolonne austretenden Kohlenwasserstoffe werden gasförmig in den Sumpf der Füllkörperkolonne zurückgeführt.

## Vergleichsversuch

In einem Vergleichsversuch verfährt man wie im vorstehenden Beispiel 1 beschrieben, wobei jedoch als selektives Lösungsmittel eine Mischung aus 91,7 Gewichtsprozent n-Methylpyrrolidon und 8,3 Gewichtsprozent Wasser verwendet wird, die der Füllkörperkolonne in einer gegenüber dem Beispiel 1 erhöhten Menge von 6,00 kg/h zugeführt wird, und das $C_4$-Kohlenwasserstoffgemisch bei gleicher Zusammensetzung wie in Beispiel 1 in einer gegenüber dem Beispiel 1 erniedrigten Menge von 0,471 kg/h am Sumpf der Füllkörperkolonne zugegeben wird, wobei 0,235 kg/h Raffinat und 0,236 kg/h Rohbutadien erhalten werden. Obwohl im Vergleichsversuch das Verhältnis von zugeführten selektiven Lösungsmittel zu zugeführtem $C_4$-Kohlenwasserstoffgemisch (12,74) gegenüber dem entsprechenden Verhältnis im Beispiel 1 (5,36) um 138% erhöht ist, wird im Vergleichsversuch bei praktisch gleichem Verhältnis der Menge an Raffinat und der Rohbutadienmenge (0,235/0,236) wie im Beispiel 1 (0,418/0,440) ein Raffinat mit einem Butadien-1,3-Gehalt von 14,77 Gewichtsprozent erhalten gegenüber einem Butadien-1,3-Gehalt im Raffinat gemäß Beispiel 1 von nur 7,89 Gewichtsprozent. Außerdem wird die Schlüsselkomponente Buten-2-cis im Raffinat gemäß dem Vergleichsversuch (4,41 Gewichtsprozent Buten-2-cis) weniger stark angereichert als im Raffinat gemäß Beispiel 1 (4,78 Gewichtsprozent Buten-2-cis). Während Butadien-1,2 Äthylacetylen und Vinylacetylen im Raffinat gemäß dem Vergleichsversuch noch deutlich nachweisbar sind, liegen die Gehalte an diesen Komponenten im Raffinat gemäß Beispiel 1 unter der Nachweisbarkeitsgrenze. Außerdem weist das im Vergleichsversuch erhaltene Rohbutadien lediglich einen Butadien-1,3-Gehalt von 69,07 Gewichtsprozent auf gegenüber einem Butadien-1,3-Gehalt im Rohbutadien gemäß Beispiel 1 von 74,55 Gewichtsprozent. Zudem wurde Buten-2-cis in Rohbutadien beim Vergleichsversuch nur auf 4,66 Gewichtsprozent abgereichert, während der entsprechende Wert beim Beispiel 1 4,09 Gewichtsprozent betrug.

Beispiel 2

Man verfährt wie im Beispiel 1 und dem zugehörigen Vergleichsversuch beschrieben, wobei jedoch im Beispiel 2 als selektives Lösungsmittel eine Mischung aus 90 Gewichtsprozent N-Methylpyrrolidon und 10 Gewichtsprozent Tetrahydrofuran verwendet und ein Verhältnis L/G von Lösungsmittelumlauf L zu Einsatz an $C_4$-Kohlenwasserstoffgemisch G von 6,50 und im Vergleichsversuch zu Beispiel 2 zur Erzielung des gleichen Trennerfolges wie im Beispiel 2 ein Verhältnis L/G von 12,74 aufrecht erhalten wird, d. h. zur Erzielung des gleichen Trennerfolges war im Vergleichsversuch gegenüber dem Beispiel ein um 96% höheres L/G-Verhältnis erforderlich.

**Patentansprüche**

1. Verfahren zur Gewinnung eines konjugierten Diolefins aus einem dieses enthaltenden $C_4$- oder $C_5$-Kohlenwasserstoffgemisch durch ein- oder mehrstufige extraktive Destillation mit einem selektiven Lösungsmittel, dadurch gekennzeichnet, daß man als selektives Lösungsmittel eine Lösungsmittelmischung verwendet, die

a) 50 bis 98 Gewichtsprozent eines N-alkylsubstituierten niederen aliphatischen Säureamids oder eines N-alkylsubstituierten alicyclischen Säureamids mit 5 Ringgliedern und
b) 2 bis 50 Gewichtsprozent eines

- aliphatischen Äthers mit einem Siedepunkt im Bereich von 30 bis 200° C der allgemeinen Formel R – O – R', worin R ein Kohlenwasserstoffrest mit 1 bis 6 Kohlenstoffatomen und R' ein Kohlenwasserstoffrest mit 2 bis 6 Kohlenstoffatomen ist, oder der allgemeinen Formel

  $$R^1-[OCH_2-CHR-]_nO-R^2,$$

  in der R ein Wasserstoffatom oder einen $CH_3$-Rest, $R^1$ und $R^2$ jeweils einen Kohlenwasserstoffrest mit 1 bis 5 Kohlenstoffatomen und n eine ganze Zahl von 1 bis 3 bedeuten, und $R^1$ darüber hinaus für ein Wasserstoffatom stehen kann, bzw.
- alicyclischen Äthers mit einem Siedepunkt im Bereich von 50° bis 125° C

enthält.

2. Verfahren nach Anspruch 1, dadurch gekennzeichnet, daß man eine Lösungsmittelmischung verwendet, die

a) 50 bis 98 Gewichtsprozent Dimethylformamid, Diäthylformamid, Dimethylacetamid, Diäthylacetamid, Formylmorpholin oder N-Methylpyrrolidon und
b) 2 bis 50 Gewichtsprozent eines

- aliphatischen Äthers mit einem Siedepunkt im Bereich von 30 bis 200° C der allgemeinen Formel R – O – R', worin R ein Kohlenwasserstoffrest mit 1 bis 6 Kohlenstoffatomen und R' ein Kohlenwasserstoffrest mit 2 bis 6 Kohlenstoffatomen ist, oder der allgemeinen Formel

  $$R^1-[OCH_2-CHR-]_nO-R^2,$$

  in der R ein Wasserstoffatom oder einen $CH_3$-Rest, $R^1$ und $R^2$ jeweils einen Kohlenwasserstoffrest mit 1 bis 5 Kohlenstoffatomen und n eine ganze Zahl von 1 bis 3 bedeuten, und $R^1$ darüber hinaus für ein Wasserstoffatom stehen kann, bzw.
- alicyclischen Äthers mit einem Siedepunkt im Bereich von 50° bis 125° C

enthält.

3. Verfahren nach Anspruch 1 und 2, dadurch gekennzeichnet, daß die Lösungsmittelmischung höchstens 5 Gewichtsprozent Wasser, bezogen auf die Lösungsmittelmischung enthält.

**Claims**

1. A process for isolating a conjugated diolefin from a $C_4$- or $C_5$-hydrocarbon mixture containing the diolefin, by single-stage or multi-stage extractive distallation using a selective solvent, characterized in that there is used as selective solvent a solvent mixture which contains

a) from 50 to 98 percent by weight of an N-alkyl-substituted lower aliphatic acid amide or of an N-alkyl-substituted alicyclic acid amide having 5 ring members and
b) from 2 to 50 percent by weight of an

- aliphatic ether, boiling at from 30° to 200° C, of the general formula R – O – R', where R is a hydrocarbon radical of 1 to 6 carbon atoms and R' is a hydrocarbon radical of 2 to 6 carbon atoms, or of the general formula

  $$R^1-[OCH_2-CHR-]_nO-R^2,$$

where R is hydrogen or $CH_3$, $R^1$ and $R^2$ are each a hydrocarbon radical of 1 to 5 carbon atoms, and n is an integer from 1 to 3, and $R^1$ may also be hydrogen, or

– alicyclic ether boiling at from 50° to 125°C.

2. A process as claimed in claim 1, characterized in that a solvent mixture is used which contains

a) from 50 to 98 percent by weight of dimethylformamide, diethylformamide, dimethylacetamide, diethylacetamide, formylmorpholine or N-methylpyrrolidone, and

b) from 2 to 50 percent by weight of an

– aliphatic ether, boiling at from 30° to 200°C, of the general formula $R-O-R'$, wehere R ist a hydrocarbon radical of 1 to 6 carbon atoms and R' ist a hydrocarbon radical of 2 to 6 carbon atoms, or of the general formula

$$R^1-[OCH_2-CHR-]_nO-R^2,$$

where R is hydrogen or $CH_3$, $R^1$ and $R^2$ are each a hydrocarbon radical of 1 to 5 carbon atoms, and n is an integer from 1 to 3, and $R^1$ may also be hydrogen, or

– alicyclic ether boiling at from 50° to 125°C.

3. A process as claimed in Claims 1 and 2, characterized in that the solvent mixture contains at most 5 percent by weight of water, based on the solvent mixture.

**Revendications**

1. Procédé d'extraction d'une dioléfine conjuguée d'un mélange d'hydrocarbures en $C_4$ ou $C_5$, qui la contient, par une destillation en un ou plusieurs stades de mélange dans un solvant sélectif, caractérisé en ce que le solvant sélectif est constitué d'un mélange de solvants, composé de:

a) 50 à 98% en poids d'un amide d'acide aliphatique inférieur substutué sur l'atome d'azote par des radicaux alcoyle ou d'un amide d'acide alicylique à noyau pentagonal, substitué sur l'atome d'azote par des radicaux alcoyle;

b) 2 à 50% en poids

– d'un éther aluphatique avec un point d'ébullition dans la gamme de 30 à 200°C, de la formule générale $R-O-R'$, das laquelle R désigne un reste hydrocarboné en $C_1$ à $C_6$ et R' un reste hydrocarboné en $C_2$ à $C_6$, ou de la formule générale

$$R^1-[OCH_2-CHR-]_nO-R^2,$$

dans laquelle R désigne un atome d'hydrogène ou un radical méthyle et $R^1$ et $R^2$ représentent chacun un reste hydrocarboné en $C_1$ à $C_5$, $R^1$ pouvant en outre représenter un atome d'hydrogène, et n est un nombre entier valant 1 à 3, ou

– d'un éther alicyclique avec un point d'ébullition dans la gamme de 50 à 125°C.

2. Procédé suivant la revendication 1, caractérisé en ce que l'on emploie un mélange de solvants, composé de:

a) 50 à 98% en poids de diméthyl-formamide, de diéthylformamide, de diméthyl-acétamide, de diéthyl-acétamide, de formylmorpholine ou de N-méthyl-pyrrolidone;

b) 2 à 50% en poids

– d'un éther aliphatique avec un point d'ébullition dans la gamme de 30 à 200°C, de la formule générale $R-O-R'$, dans laquelle R désigne un reste hydrocarboné en $C_1$ à $C_6$ et R' un reste hydrocarboné en $C_2$ à $C_6$, ou de la formule générale

$$R^1-[OCH_2-CHR-]_nO-R^2,$$

dans laquelle R désigne un atome d'hydrogène ou un radical méthyle et $R^1$ et $R^2$ représentent chacun un reste hydrocarboné en $C_1$ à $C_5$, $R^1$ pouvant en outre représenter un atome d'hydrogène, et n est un nombre entier valant 1 à 3, ou

– d'un éther alicyclique avec un point d'ébullition dans la gamma de 50 à 125°C.

3. Procédé suivant l'une des revendications 1 et 2, caractérisé en ce que le mélange de solvants contient au maximum 5% en poids d'eau par rapport au poids du mélange de solvants.